(19) **Europäisches Patentamt**

**European Patent Office**

**Office européen des brevets**

(11) **EP 1 067 880 B1**

(12) **EUROPÄISCHE PATENTSCHRIFT**

(45) Veröffentlichungstag und Bekanntmachung des Hinweises auf die Patenterteilung:
**01.10.2003 Patentblatt 2003/40**

(51) Int Cl.⁷: **A61C 13/00**

(21) Anmeldenummer: **99939826.6**

(22) Anmeldetag: **16.03.1999**

(86) Internationale Anmeldenummer:
**PCT/CH99/00115**

(87) Internationale Veröffentlichungsnummer:
**WO 99/047065 (23.09.1999 Gazette 1999/38)**

(54) **ZAHNKRONEN UND/ODER ZAHNBRÜCKEN**

DENTAL CROWNS AND/OR DENTAL BRIDGES

COURONNES DENTAIRES ET/OU BRIDGES DENTAIRES

(84) Benannte Vertragsstaaten:
**AT BE CH DE DK ES FI FR GB IE IT LI LU NL PT SE**

(30) Priorität: **17.03.1998 EP 98810230**
**12.11.1998 EP 98811131**

(43) Veröffentlichungstag der Anmeldung:
**17.01.2001 Patentblatt 2001/03**

(73) Patentinhaber: **Eidgenössische Technische Hochschule Zürich Nichtmetallische Werkstoffe 8092 Zürich (CH)**

(72) Erfinder:
• **FILSER, Frank**
**CH-8102 Oberengstringen (CH)**
• **GAUCKLER, Ludwig**
**CH-8200 Schaffhausen (CH)**
• **KOCHER, Peter**
**CH-8304 Wallisellen (CH)**
• **LUETHY, Heinz**
**CH-2000 Neuchâtel (CH)**
• **SCHAERER, Peter**
**CH-8053 Zürich (CH)**

(74) Vertreter: **Patentanwälte Breiter + Wiedmer AG Seuzachstrasse 2 Postfach 366 8413 Neftenbach/Zürich (CH)**

(56) Entgegenhaltungen:
**EP-A- 0 160 797** **EP-A- 0 580 565**

EP 1 067 880 B1

**Beschreibung**

Die Erfindung bezieht sich auf ein Verfahren zur Herstellung von auf wenigstens einen vorpräparierten Zahnstumpf aufpassbarem künstlichem Zahnersatz nach dem Oberbegriff von Patentanspruch 1. Weiter betrifft die Erfindung einen Rohling aus poröser Keramik zur Durchführung des Verfahrens.

Es sind zahlreiche Methoden zur Herstellung von künstlichen Zahnkronen und/oder Zahnbrücken bekannt. Grundsätzlich wird nach der zahnärztlichen Präparation ein Abdruck des Zahnstumpfes/der Zahnstümpfe, der Zahnumgebung und des Kiefers angefertigt. Manuell kann über Gipsabformung ein Positivmodell der Situation im Mund hergestellt werden, auf dem mit handwerklichem Geschick ein Grundgerüst in Wachs oder Kunststoff modelliert wird. Bei Anwendung der konventionellen Technik wird über bekannte Verfahren, wie das Wachsausschmelzverfahren, 1:1 Kopierfräsen oder -schleifen, ein Modell des Grundgerüstes in Metall ausgeführt und mit Porzellan überbrannt. Neben dem hohen Ausschussrisiko beim Aufbrennen des Porzellans sind Abstriche in ästhetischer Hinsicht, vorallem am zervikalen Rand, hinzunehmen und röntgenbasierte Diagnoseverfahren zur Überwachung der überkronten Zähne nicht mehr anwendbar. In anderen Verfahren eingesetzte sogenannte dentalkeramische Porzellane sind aufgrund ihrer schlechten mechanischen Eigenschaften wohl für niedrig belastete Zahnkronen geeignet, für Zahnbrücken dagegen nicht einsetzbar.

In der EP, B1 0389461 wird ein Verfahren zur Herstellung eines künstlichen Zahnkronen-Onlays (onlay tooth crown) zum Einpassen in eine vorbereitete Zahnkavität beschrieben. Das Verfahren geht von einem Abdruck oder einer Negativform der Situation im Patientenmund aus. Die Zahnkronen-Onlays werden durch Kopierfräsen aus einem grünen oder vorgesinterten keramischen Rohling vergrössert herausgefräst und anschliessend dicht gesintert. Die Zahnkronen-Onlays gemäss der EP,B1 0389461 sind von Zahnkronen und Zahnbrücken grundsätzlich verschiedene Produkte, die zahnmedizinische Indikation ist eine andere. Zahnkronen-Onlays werden in Zahnhöhlungen eingepasst und sind bezüglich der geometrischen Form stets konvex geformt. Zahnkronen, auch mit Zahnbrücken, werden auf einen Zahnstumpf aufgepasst und haben die Form eines Käppchens. Dadurch ergeben sich dünn auslaufende Berandungen, welche technologisch schwierig zu handhaben sind. Ein wesentliches Merkmal des Kopierfräsens gemäss der EP,B1 0389461 ist die Zeitgleichheit von Abtasten und Übertragen der Abtastbewegung auf ein Bearbeitungswerkzeug. Im wesentlichen entspricht dies der Arbeitsweise eines Pantographen, welcher seit langem für lineare Zeichnungsvergrösserungen eingesetzt wird. Der Anwendungsbereich der EP, B1 0389461 ist deshalb auf ausschliesslich konvex geformte Zahnersatzprodukte beschränkt, wie beispielsweise Inlays, Onlay-Zahnkronen und Verblendungen.

Die EP,A2 580565 beschreibt künstliche Zahnrestaurationen mit pulvermetalurgischen Herstellungsverfahren eines keramisch dichten, hochfestem Grundgerüstes, welches mit Dentalporzellan beschichtet wird. Die Form der Zahnpräparation wird im Mund oder an einem Zahnmodell optisch oder mechanisch aufgenommen. Die Kavitäten, d.h die innere Oberfläche, und ihre lokale Beziehung zueinander werden vergrössert aus einem Material, z.B. aus Kunststoff, mit einer rechnergesteuerten Fräsmaschine hergestellt. Die Kavitäten des Grundgerüstes werden in einem pulvermetallurgischen Prozess mit dieser Form hergestellt, also durch Pressen von Pulver über einen vorgefertigten, vergrösserten Zahnstumpf. Die äussere Oberfläche des Grundgerüstes wird ebenfalls durch Pressen gestaltet. Das Verfahren zur Herstellung des Grundgerüstes unterscheidet sich also grundsätzlich vom Herausarbeiten aus einem Rohling durch Materialabtrag.

Schliesslich ist aus der EP,A1 0160797 ein Rohling zur Herstellung zahntechnischer Formteile bekannt. Der zu bearbeitende Rohlingskörper weist einen eng tolerierten Abschnitt auf, welcher auch als Ansatzstück ausgebildet sein kann. Dieses mit Referenzflächen bzw. -anschlägen versehene Ansatzstück dient als Halter zum Einsetzen des Rohlings in eine Spannzange für die materialabhebende Bearbeitung. Die Referenzflächen können von der Bearbeitungsmaschine abtastbare, codierte Information über die Rohlingseigenschaften enthalten.

Die Erfinder haben sich die Aufgabe gestellt, ein Verfahren der eingangs genannten Art zu schaffen, welches die Herstellung von vollkeramischen Zahnkronen und/oder Zahnbrücken mit einem Grundgerüst aus dicht gesinterter, hochfester Keramik zum Aufpassen und adhäsiven und/oder retentiven Befestigen auf natürlichen oder künstlichen Zahnstümpfen erlaubt. Das Verfahren soll die Herstellung von Zahnkronen und/oder Zahnbrücken mit okklusaler und kavitaler Oberfläche aus beim Sintern schrumpfenden Werkstoffen erlauben, welche auch bei filigraner Gestalt eine einwandfreie Passform haben, also keine Nacharbeit erforderlich machen. Weiter soll ein Rohling aus oxidkeramischem Material zur Verfügung stehen, der eine einfache, exakte Durchführung des Verfahrens erlaubt

Die Aufgabe wird in bezug auf das Verfahren erfindungsgemäss nach dem Kennzeichen von Patentanspruch 1 gelöst. Spezielle und weiterbildende Ausführungsformen des erfindungsgemässen Verfahrens sind Gegenstand von abhängigen Ansprüchen.

Ausgehend von einer zahnärztlichen Präparation der Zahnstümpfe wird ein Abdruck angefertigt, der ein Negativmodell der Situation im Mund des Patienten abformt, insbesondere die Oberfläche des Zahnstumpfes oder der Zahnstümpfe, die Approximalflächen der Nachbarzähne und der Gegenbiss. Weitergehend von dieser Abformung wird ein Positivmodell, meist aus Gips, hergestellt. Auf dem Positivmodell der Situation wird Abstandslack aufgetragen, welcher einen Spalt zwischen den Oberflächen des aufgrund des Modells hergestellten Grundgerüstes und des Zahnstumpfes berücksichtigt. Darauf kann auf dem bezeichneten Positivmodell der Situation im Patientenmund ein Modell für das Grundgerüst aus Wachs oder Kunststoff angefertigt werden. Dieses Vorgehen ist bekannt und wird in der zahntechnischen Praxis auch zur Herstellung von metallischen Grundgerüsten für Zahnkronen und/oder Zahnbrücken angewendet.

Das erfindungsgemässe Verfahren schliesst an diese an sich bekannte Vorstufe an und digitalisiert die äussere und innere Oberfläche des Grundgerüstmodells bzw. die Oberflächen am Positivmodell vollständig. Ein die Situation im Patientenmund unvollständig wiedergebendes Positivmodell wird vorzugsweise bezüglich der dreidimensionalen äusseren und inneren Oberfläche rechentechnisch ergänzt, was insbesondere im Bereich der Brückenglieder von Zahnbrücken von Bedeutung ist. Das Ergebnis der Digitalisierung und einer allfälligen rechentechnischen Ergänzung ist eine digitale Beschreibung der kompletten Oberfläche des Grundgerüstes. Die Digitalisierung kann mechanisch oder optisch erfolgen. Verfahren zur Digitalisierung im Mund eines Patienten auf einem präparierten Zahnstumpf oder an einem Modell sind beispielsweise aus der US,A 4182312 (mechanisch) und EP,B1 0054785 (optisch) bekannt. Der wesentliche Nachteil des bekannten mechanischen Digitalisierens liegt in der Fixierung des mechanischen Abtastgerätes am Patienten, die sichere Handhabung des Gerätes in der engen Mundhöhle ist problematisch. Bei optischen Digitalisiergeräten ist es notwendig, den Zahnstumpf aufgrund seiner transluszenten Eigenschaften mit Pulver zu beschichten, um Ungenauigkeiten durch teilweises und unkontrolliertes Eindringen von Licht in den zu vermessenden Zahnstumpf zu verhindern. Die Beschichtung mit einer Pulverschicht erhöht aber gleichzeitig auch die Ungenauigkeit durch das Auftragen einer meist ungleichmässigen Pulverhöhe auf den Zahnstumpf.

Im erfindungsgemässen Verfahren kann das Grundgerüstmodell mit Klemmstiften aufgespannt werden. Das aufgespannte Grundgerüstmodell wird zweckmässig in Schritten gedreht. Eine Drehung um 180° erlaubt eine vollständige Digitalisierung der okklusal und kavital zugänglichen Oberflächen des Grundgerüstmodells. Die optimalen Arbeitspositionen werden vorausbestimmt und durch Drehen der Wellen angesteuert.

Die Dimensionen der Oberfläche des Grundgerüstmodells werden zur Kompensation der Schrumpfung beim Sintern in allen Raumrichtungen linear vergrössert. Der Vergrösserungsfaktor f ergibt sich aus dem Raumgewicht $\rho_R$ des vorgefertigten Rohlings und dem erreichbaren Raumgewicht $\rho_S$ nach der Sinterung gemäss Gleichung 1

$$f = 3\sqrt{\frac{\rho_S}{\rho_R}} \qquad (1)$$

Aus den Daten der vergrösserten Oberfläche werden die Steuerbefehle für die Bearbeitungsmaschine generiert, mit denen das vergrösserte Grundgerüst aus dem Rohling vollständig und vergrössert herausgearbeitet wird. Gegenüber der vergrösserten Oberfläche des Grundgerüstmodells wird kein Aufmass belassen, so dass bei der nachfolgenden Sinterschrumpfung direkt die exakten Endmasse erreicht werden, wodurch eine Nachbearbeitung im dicht gesinterten Zustand vermieden wird.

Zeitlich entkoppelt von der Digitalisierung kann nun ein Rohling aus poröser Keramik durch Materialabtrag zum vergrösserten Grundgerüst geformt werden. Dazu kann der Rohling z.B. zwischen zwei Wellen der Bearbeitungsmaschine aufgespannt werden. Nach einem abgeleiteten geeigneten Werkzeugweg wird der drehbar gelagerte Rohling bearbeitet. Die Bearbeitung kann mechanisch, beispielsweise mittels der Fertigungsverfahren Fräsen oder Schleifen mit einem oder mehreren Werkzeugen, und/oder optisch mit einem oder mehreren Strahlen erfolgen, beispielsweise durch Laserstrahlen. Die Bearbeitung kann in einem oder in verschiedenen Bearbeitungsschritten erfolgen, beispielsweise zuerst eine Rohbearbeitung, dann die Endbearbeitung der vom Werkzeug zugänglichen Oberflächen. Beim Wechsel von okklusaler zu kavitaler Bearbeitung kann ein Positionswechsel des teilbearbeiteten Rohlings notwendig sein. Das Drehen der Wellen mit dem gehalterten Rohling kann programmgesteuert schrittweise und/oder kontinuierlich erfolgen, mit insgesamt einer halben, einer ganzen oder mehreren Umdrehungen, auch mit Rückwärtsdrehungen.

Der Materialabtrag von einem Rohling erfolgt vorzugsweise mit Fräserwerkzeugen mit geometrisch bestimmten Schneiden, bei Drehzahlen im Bereich von vorzugsweise 10'000 bis 50'000 Touren/min., einer Zustellung von vorzugsweise > 0,5 mm, insbesondere von 1 - 15 mm, und einer Vorschubgeschwindigkeit von vorzugsweise > 3 cm/sec, insbesondere 3,5 -10 cm/sec.

Die Herstellung des gegenüber dem Positivmodells vergrösserten Grundgerüstes aus dem Werkstoff des Rohlings wird das distale oder mesiale Abtrennen des Grundgerüstes von den Resten des Rohlings abgeschlossen. An den Trennstellen kann eine geringe manuelle Nacharbeit, ein sogenanntes Verputzen, notwendig sein.

Das fertig bearbeitete vergrösserte Grundgerüst wird dicht gesintert. Je nach verwendetem Material und Pulvermorphologie bewegen sich die Temperaturen in der Regel im Bereich von 1100 bis 1600°C. So kann eine Dichte von 90 bis 100% der theoretisch möglichen Dichte, vorzugsweise eine Dichte von 96 bis 100% der theoretisch möglichen Dichte, insbesondere mehr als 99% der theoretisch erreichbaren Dichte erreicht werden. Während des Sinterns schrumpft das Grundgerüst linear, ohne weitere Verformung oder Verzug. Dies erlaubt, den Sinterbrand ohne einen mitschwindenden Sinterstumpf durchzuführen. Die Schrumpfung S berechnet sich entsprechend der Gleichung (1) aus dem Raumgewicht des Rohlings $\rho_R$ vor der Sinterung und dem erreichbaren Raumgewicht $\rho_S$ nach der Sinterung:

$$S = 3 \sqrt{\frac{\rho_R}{\rho_S}} - 1 \qquad (2)$$

Nach der Sinterung wird das geschrumpfte keramische Grundgerüst nach herkömmlichen Aufbrennverfahren bei Temperaturen von 700 bis 1100°C mit einer Beschichtung aus Porzellan, oder mit einer Beschichtung aus Kunststoff versehen. Es können eine oder mehrere Schichten aus Porzellan oder Kunststoff aufgebracht werden. Damit erhält die Zahnkrone oder Zahnbrücke ein individuelles Aussehen. Abschliessend wird die Zahnkrone oder Zahnbrücke mit Zement auf dem präparierten Zahnstumpf befestigt, wobei herkömmliche Zementierungswerkstoffe und Vorbereitungsprozeduren angewendet werden.

Die Vorteile des erfindungsgemässen Verfahrens können wie folgt zusammengefasst werden:

- Mit einem kostengünstigen, einfachen und sicheren Verfahren können qualitativ hochwertige und massgenaue, dicht gesinterte vollkeramische Zahnkronen und/oder Zahnbrücken hergestellt werden. Für ein sicheres und einfaches Herstellungsverfahren bilden homogene Rohlinge eine wesentliche Voraussetzung.

- Die individualisierten Zahnkronen und/oder Zahnbrücken zum Aufpassen auf präparierte Zahnstümpfe widerstehen den hohen Belastungen im Seitenzahnbereich und erfüllen die ästhetischen Ansprüche des Patienten im Frontzahnbereich gleichermassen. Insbesondere im Fall von Zahnbrücken besteht das Ziel in einer hohen Separation, d. h. mit einer grazilen Gestaltung zwischen den Brückengliedern kann eine mit metallkeramischen Zahnbrücken wenigstens vergleichbare Gestaltung, die von Zahnärzten aus ästhetischen, hygienischen und phonetischen Gründen gefordert wird, erreicht werden.

Bezüglich des Rohlings aus poröser Keramik wird die Aufgabe erfindungsgemäss dadurch gelöst, dass auf dem Rohling selbst, seiner Verpackung, einer Anhängeetikette oder einem Beipackzettel ein maschinell oder mit menschlichen Sinnesorganen erfassbarer Identifikationscode mit Daten zur individuellen Eingabe des kompensierenden Vergrösserungsfaktors f aufgebracht ist.

Poröse Rohlinge aus Keramik für die Herstellung von Grundgerüsten für Zahnkronen und/oder Brücken können aus verschiedensten Materialkompositionen bestehen, insbesondere aus wenigstens einem Metalloxidpulver der Gruppe bestehend aus $Al_2O_3$, $TiO_2$, MgO, $Y_2O_3$ und Zirkonoxidmischkristall $Zr_{1-x}Me_xO_2\text{-}(\frac{4n}{2})_x$ bestehen, wobei Me ein Metall ist, das in Oxidform als 2-, 3- oder 4-wertiges Kation vorliegt (n = 2, 3, 4 und $0 \leq x \leq 1$) und die tetragonale und/oder kubische Phase des Zirkonoxids stabilisiert. Weitere Details der Materialkomposition von Rohlingen ergeben sich aus den abhängigen Verfahrensansprüchen 11 bis 13. Die Rohlinge können auch eine thermische Vorbehandlung erfahren, welche in den abhängigen Verfahrensansprüchen 6 bis 10 näher erläutert sind.

In jedem der Verfahrensschritte zur Herstellung eines Rohlings bestehen Toleranzen, z.B. Temperaturprofile und Temperaturschwankungen während der thermischen Vorbehandlung eines Rohlings.

Der Vergrösserungsfaktor f (Gleichung 1) für die Herausarbeitung der Grundgerüste aus Rohlingen ist aus den vorgenannten Gründen normalerweise keine Konstante. Auch wenn die Rohlinge aus ein- und demselben Material bestehen und auf denselben Fertigungseinrichtungen mit demselben Verfahren hergestellt werden, bleibt der Vergrösserungsfaktor f nicht konstant. Erfindungsgemäss können die Flexibilität im Material und die Fertigungstoleranzen erreicht werden, indem der individuelle Vergrösserungsfaktor f für jeden Rohling bestimmt und zusammen mit jedem Rohling ausgeliefert wird. Dies wird vorzugsweise dadurch realisiert, dass die Daten für den Vergrösserungsfaktor f optisch, elektromagnetisch oder mechanisch-taktil erfassbar auf dem Rohling selbst, seiner Verpackung, einer Anhängeetikette oder einem Beipackzettel aufgebracht ist.

Nach der einfachsten Variante sind die Daten für den Hersteller von Zahnkronen und/oder -brücken von Auge lesbar und können direkt oder über ein Hilfsprogramm zur Herstellung einer vergrösserten Ausführungsform eines Positivmodells für ein Grundgerüst ausgewertet werden.

Vorzugsweise wird jedoch ein an sich bekanntes Identifikationssystem eingesetzt, mit welchem die Daten für den Vergrösserungsfaktor f eingelesen und automatisch in Steuerbefehle für Werkzeuge umgewandelt werden.

## Ausführungsbeispiele

Ein Grundgerüst für eine Zahnbrücke zum Aufpassen auf eine zahnärztliche Präparation wird aus tetragonal stabilisiertem $ZrO_2$-Pulver hergestellt, welches 5,1 gew% $Y_2O_3$ und geringe Verunreinigungen von insgesamt weniger 0,05 gew% an $Al_2O_3$, $SiO_2$, $Fe_2O_3$ und $Na_2O$ enthält. Die Primärpartikelgrösse ist submikron, sie liegt bei etwa 0,3 μm. Die Presslinge werden isostatisch bei etwa 300 MPa gepresst und im Grünzustand wird die äusserste Materialschicht von weniger als 2 mm Dicke durch Drehen entfernt. Nach der Vorbearbeitung liegt der Durchmesser bei 22 mm und die Höhe bei 47 mm. Das Raumgewicht wird mit 3.185 g/cm$^3$ bestimmt.

Der bearbeitete Pressling wird während etwa 120 min bei etwa 850°C vorgesintert. Nach dem Ausbrennen der Bindersubstanzen wird das Raumgewicht 3.089 g/cm³ nach der Vorsinterung ermittelt.

Eine Abformung der Situation im Mund eines Patienten wird mit Siliconmasse angefertigt, insbesondere wird eine Negativform der präparierten Zahnstümpfe mit der Präparationsgrenze und den Approximatflächen der Nachbarzähne erzeugt. Durch die Abformung in einer Gipsmasse wird eine Positivform hergestellt. Zur Erzeugung eines Zementspaltes werden die beiden präparierten Zahnstümpfe auf der Oberfläche mit Distanzlack gleichmässig bestrichen und so die formgebende Oberfläche gebildet. Auf dieser Positivform der Situation im Mund des Patienten wird das Grundgerüst aus Wachs im Positiv modelliert.

Das Wachsmodell des Grundgerüstes wird zwischen zwei Wellen aufgespannt und dann vorerst schlangenlinienähnlich die okklusal zugänglichen Oberflächen, anschliessend - nach der Drehung des Wachsmodells um 180° - die kavital zugänglichen Oberflächen mechanisch digitalisiert. Das Ergebnis ist eine digitale Beschreibung der vollständigen Oberfläche des Grundgerüstes.

Die digitale Beschreibung wird linear um den gemäss Formel (1) berechneten Vergrösserungsfaktur f = 1.2512 gedehnt und davon Steuerungsbefehle für die Bearbeitungsmaschine unter Berücksichtigung der verwendeten Bearbeitungswerkzeuge für eine Grob- und eine Endbearbeitung des Grundgerüstes generiert und an die Bearbeitungsmaschine transferiert. Die Schaftfräswerkzeuge mit runder Stirnfläche haben einen Durchmesser von 3 mm für die Grobbearbeitung und einen Durchmesser von 1,5 mm für die Endbearbeitung. Der zwischen zwei Wellen gespannte, teilbearbeitete Rohling wird um 180° gedreht, so dass die Oberfläche des Grundgerüstes vollständig und vergrössert aus dem Rohling herausgearbeitet werden kann. Dann wird das Grundgerüst vom Restrohling getrennt, die Abtrennstellen am Grundgerüst durch Schleifen angeglichen und das Grundgerüst von Pulverresten sorgfältig gesäubert.

Das vergrösserte Grundgerüst aus $ZrO_2$ mit $Y_2O_3$ wird dann bei etwa 1500° C gesintert. Nach dem Sintern wird ein Raumgewicht von 6,050 g/cm³ ermittelt, was praktisch 100% des maximal erreichbaren Raumgewichtes entspricht. Das bei der Sinterung um 20,07% geschrumpfte Grundgerüst kann ohne weitere Nachbearbeitung auf dem Positivmodell der Situation im Mund des Patienten aufgepasst werden.

Abschliessend wird das Grundgerüst durch Aufbrennen von Schichten von Porzellan bei Temperaturen zwischen 700 und 1100°C individualisiert und im Mund des Patienten mit Phosphatzement adhäsiv befestigt.

Anhand von in der Zeichnung dargestellten Ausführungsbeispielen, welche auch Gegenstand von abhängigen Patentansprüchen sind, wird die Erfindung näher erläutert. Es zeigen schematisch:

- Fig. 1   einen Längsschnitt durch einen natürlichen Zahnstumpf mit einer künstlichen Zahnkrone,
- Fig. 2   ein vergrössertes Detail von Bereich A gemäss Fig. 1,
- Fig. 3   einen Längsschnitt durch zwei Zahnstümpfe mit einer dreigliedrigen Zahnbrücke,
- Fig. 4   eine okklusale Ansicht des Grundgerüstes einer Zahnbrücke,
- Fig. 5   eine kavitale Ansicht des Grundgerüstes einer Zahnbrücke,
- Fig. 6   die Aufspannsituation eines Grundgerüstmodells für das Digitalisieren,
- Fig. 7   die Aufspannsituation für einen unbearbeiteten Rohling,
- Fig. 8   die Aufspannsituation vor dem Heraustrennen eines herausgearbeiteten Rohlings, und
- Fig. 9   die Aufspannsituation für das Digitalisieren eines Grundgerüstmodells einer Zahnkrone.

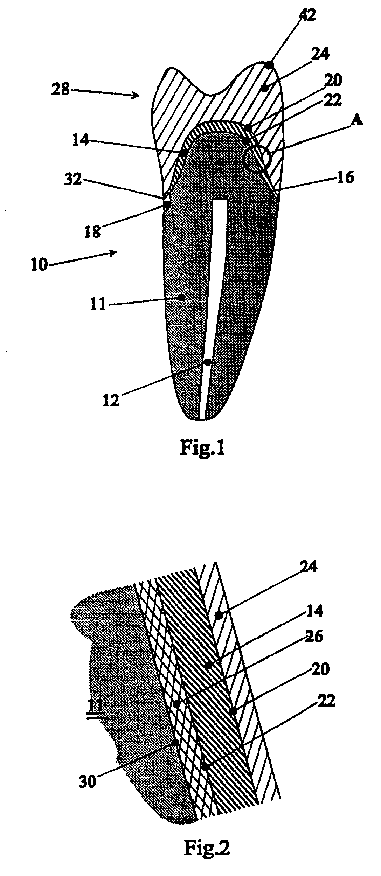

Ein in Fig. 1 dargestellter Zahnstumpf 10 hat eine Pulpa 12 für einen nicht dargestellten Nerv. Dieser Zahnstumpf ist natürlich und vital, nach anderen Ausführungsformen kann der Zahnstumpf 10 natürlich und avital oder künstlich auf einem Implantat aufgebaut sein. Der Zahnstumpf 10 weist keine Hinterschneidungen auf.

Auf den Zahnstumpf 10 ist ein Grundgerüst 14 aus dicht gesintertem Keramikmaterial aufzementiert. Dieses Grundgerüst 14 weist eine in Richtung eines Enamels 18 dünn auslaufende Berandung 16 auf, welche wesentlich schwieriger und filigraner herzustellen ist, als eine bekannte Onlay-Zahnkrone mit ausschliesslich konvexen Flächen. Die äussere Oberfläche 20 des Grundgerüstes 14 verläuft konvex und kann okklusal bearbeitet werden, was weitgehend dem Stand der Technik entspricht. Die konkave innere Oberfläche 22 des Grundgerüstes 14 wird kavital bearbeitet, was insbesondere mit Blick auf die dünn auslaufende Berandung 16 äusserst heikel ist. Mit der vorliegenden Erfindung kann dieses Problem auch beim Einsatz von vollkeramischem Material gelöst werden.

Zur Bildung einer künstlichen Zahnkrone wird Beschichtungsmaterial 24 auf das Grundgerüst 14 aufgetragen, bis die ursprünglich natürliche Zahnform wieder hergestellt ist. Mit dem Beschichtungsmaterial 24 wird das Grundgerüst 14 individualisiert, d.h. mit Porzellan oder Kunststoff verblendet.

Im vergrösserten Bereich A gemäss Fig. 2 ist deutlich erkennbar, dass beidseits des Grundgerüstes 14 aus dicht gesinterter Keramik weitere Schichten ausgebildet sind. In Richtung des Dentins 11 ist eine Zementschicht 26 zur adhäsiven Befestigung des Grundgerüstes 14 auf dem Zahnstumpf 10. Das Beschichtungsmaterial 24 ist lediglich als verhältnismässig dünne Schicht eingezeichnet, diese Schicht kann wesentlich dicker und mit formender Aussenfläche 42 ausgestaltet sein und so eine Zahnkrone 28 bilden.

Die Oberfläche 30 des natürlichen Zahnstumpfs 10 wird durch die zahnärztliche Präparation gebildet. Diese Oberfläche 30 verläuft bis zur Präparationsgrenze 32, auf welcher die dünn auslaufende Berandung 16 des Grundgerüstes 14 aufliegt.

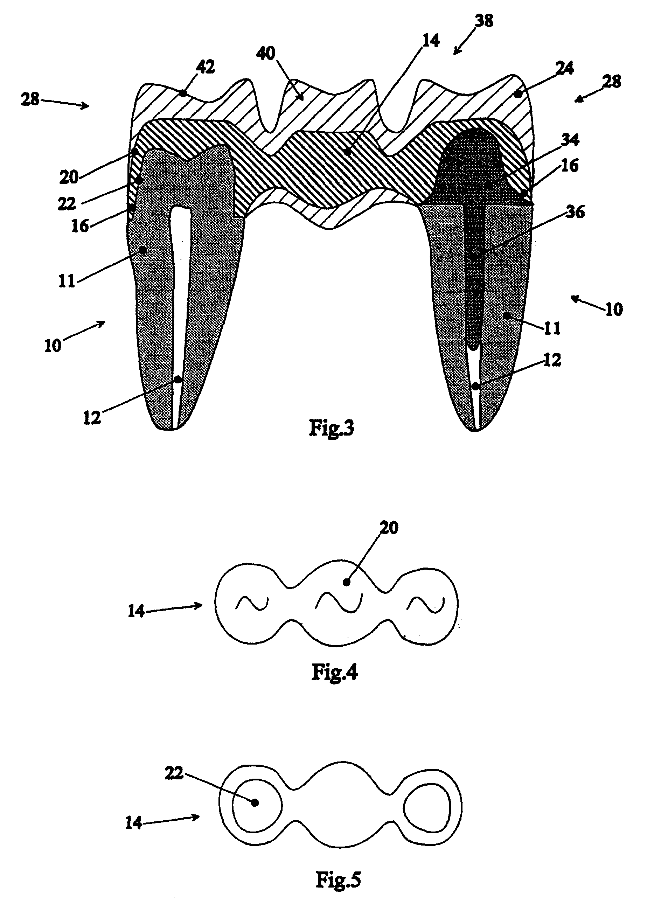

Ein links gezeichnete Zahnstumpf 10 gemäss Fig. 3 entspricht weitgehend demjenigen von Fig. 1. Ein auf der rechten Seite von Fig. 3 gezeichneter avitaler Zahnstumpf 10 hat einen unteren Rest von Dentin 11, auf welchen ein künstlicher Zahnstumpf 34 aufgesetzt und in der leblosen Pulpa 12 über einen Stift 36 verankert ist. Auf die beiden Zahnstümpfe 10 ist eine dreigliedrige Zahnbrücke 38 mit einer Zementschicht 26 (Fig. 2) adhäsiv befestigt. Diese Zahnbrücke 38 umfasst zwei Zahnkronen 28 und ein Brückenglied 40, sie dient als Ersatz von verlorener Zahnsubstanz. Auf einem ebenfalls dreigliedrigen Grundgerüst 14 aus hochfester, dicht gesinterter Keramik ist durch Verblenden mit Beschichtungsmaterial 24 aus Porzellan oder Kunststoff individualisiert. Dieses Beschichtungsmaterial hat eine Aussenfläche 42, welche möglichst derjenigen der ursprünglichen natürlichen Zähne entspricht.

Nach einer nicht dargestellten Ausführungsform kann eine Zahnbrücke 38 mehr als zwei unterstützende Zahnstümpfe 10 und/oder mehrere Brückenglieder 40 haben. Wie bereits erwähnt, können die unterstützenden Zahnstümpfe 10 auch Implantate mit künstlichen Zahnstümpfen sein.

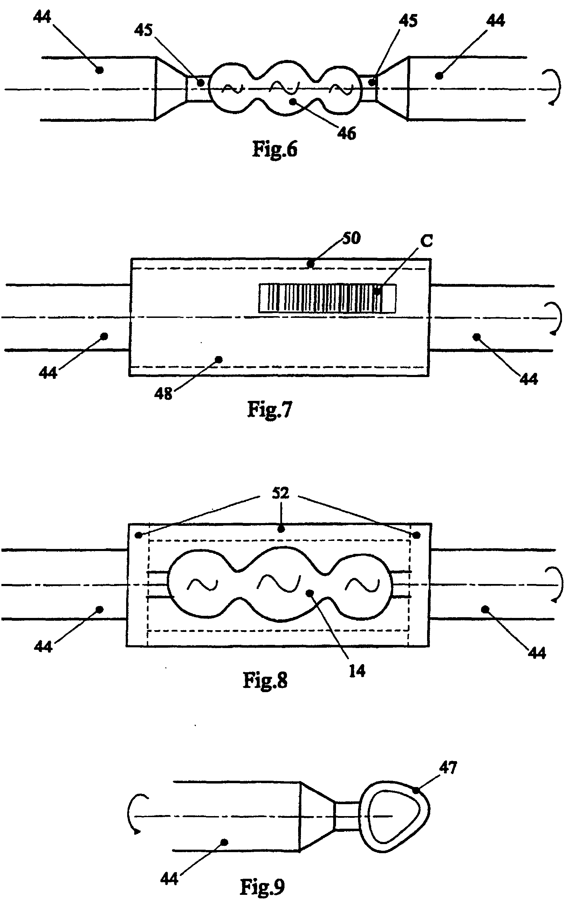

In der okklusalen Ansicht gemäss Fig. 4 ist ein dreigliedriges Grundgerüst 14 mit der äusseren konvexen Oberfläche 20 dargestellt, in Fig. 5 eine kavitale Ansicht dieses Grundgerüstes 14 einer Zahnbrücke mit der inneren konkaven Oberfläche 22.

Fig. 6 zeigt zwei coaxiale, synchron angetriebene Wellen 44 mit je einer stirnseitigen Verengung zu einem Klemmstift 45. Eingespannt ist ein dreigliedriges Grundgerüstmodell 46 einer Zahnbrücke 38 (Fig. 3) in okklusaler Ansicht. Die Wellen 44 mit den stirnseitigen Klemmstiften 45 sind in axialer Richtung verschiebbar und um einstellbare Winkel synchron drehbar. Nach dem Digitalisieren der okklusalen Seite des Grundgerüstmodells 46 werden die Wellen 44 um 180° gedreht und auch die kavitale Seite digitalisiert.

In Fig. 7 ist ein zwischen zwei Wellen 44 drehbar eingespannter Rohling 48 aus gepresstem Keramikpulver dargestellt. Direkt auf dem Rohling 48 ist ein maschinenlesbarer Informationscode C mit Daten für den Vergrösserungsfaktor f aufgebracht, im vorliegenden Fall ein elektromagnetisch oder optisch lesbarer Strichcode. Der Informationscode C dient beispielsweise der Identifikation.

Für die Herstellung des Rohlings 48 werden Pulver oder Kolloide über bekannte Methoden der keramischen Formgebung zu grünen Rohlingen verarbeitet. Bekannte Verfahren zur Herstellung keramischer Grünkörper sind beispielsweise in der WO 94/02429 und 94/24064 beschrieben. Aus fertigungstechnischen Gründen werden für die Rohlinge geometrisch einfache Gestaltformen, wie Zylinder oder Quader, bevorzugt.

Vor der Vorbearbeitung kann der Rohling 48 einer Wärmebehandlung unterzogen werden. Diese erfolgt bevorzugt bei Temperaturen im Bereich von 50 bis 200°C, insbesondere von 90 bis 150°C, für eine Zeitdauer von bevorzugt 2 bis 20 Stunden, insbesondere von 2 bis 6 Stunden. Unmittelbar anschliessend kann der Rohling 48 materialabtragend zum vergrösserten Grundgerüst 14 weiterverarbeitet werden.

Die äussere Schicht 50 wird insbesondere abgetragen, wenn der Rohling 48 über Press-, Giess- oder Spritzverfahren hergestellt ist, um vorhandene Dichtegradienten in der äussersten Materialhülle abzutragen. Weitere übliche Herstellungsverfahren für Rohlinge 48 sind kaltisostatisches Pressen, uniaxiales Pressen, Schlickerguss, Druckguss, Spritzguss, Extrudieren, Rollen und DCC (direct coagulation casting).

Vor dem Weiterverarbeiten zum vergrösserten Grundgerüst 14 kann der Rohling 48 eine Vorsinterung erfahren, welche vorzugsweise während 0,5 bis 6 Stunden bei einer Temperatur von mindestens etwa 450°C, insbesondere in einem Temperaturbereich von 600 bis 1200° C, durchgeführt wird.

Die Rohlinge werden in der Praxis meist aus einem Metalloxidpulver der Gruppe bestehend aus $Al_2O_3$, $TiO_2$, $MgO$, $Y_2O_3$ und Zirkonoxidmischkristall $Zr_{1-x}Me_xO_2 \cdot (\frac{4n}{2})_x$ hergestellt, wobei Me ein Metall ist, das in Oxidform als zwei-, drei- oder vierwertiges Kation vorliegt und die tetragonale und/oder die kubische Phase des Zirkonoxids stabilisiert. Bei der Formel für Zirkonmischoxidkristall ist n = 2, 3 oder 4, weiter gilt $0 \leq x \leq 1$.

Nach einer speziellen Ausführungsform wird das Metalloxidpulver mit einem organischen Binder vermischt, vorzugsweise aus wenigstens einer der Klassen Polyvinylalkohole (PVA), Polyacrylsäuren (PPA), Zellulosen, Polyethylenglukole und/oder Thermoplaste. Zweckmässig liegt der Binderanteil im Bereich von 0,1 bis 45 vol%, vorzugsweise im Bereich von 0,1 bis 5vol%.

Fig. 8 zeigt in okklusaler Ansicht die Aufspannsituation nach der Bearbeitung, jedoch vor dem Heraustrennen des vergrösserten Grundgerüstes 14 von den verbliebenen Resten 52 des Rohlings 48.

In Fig. 9 wird in kavitaler Ansicht die Aufspannsituation für das Digitalisieren eines Grundgerüstmodells 47 für eine Zahnkrone gezeigt.

**Patentansprüche**

1. Verfahren zur Herstellung von auf wenigstens einen vorpräparierten Zahnstumpf (10) aufpassbarem künstlichem Zahnersatz (28,38) aus gepresstem, feinem Keramikpulver, wobei unter Berücksichtigung der Schrumpfung die innere Oberfläche (22) eines vollkeramischen Grundgerüsts (14) aus bio-

logisch verträglichem Material berechnet wird, indem die geometrischen Verhältnisse des Patientenmundes abgetastet und digitalisiert, die Daten um einen die Sinterschrumpfung exakt kompensierenden Vergrösserungsfaktor (f) in allen Richtungen linear vergrössert, in die Steuerelektronik wenigstens einer Bearbeitungsmaschine übertragen und davon geeignete Werkzeugwege abgeleitet werden, die vergrösserte Ausführungsform des Grundgerüsts (14) auf die direkten Endmasse dichtgesintert und dann durch Verblenden mit Beschichtungsmaterial (24) aus Porzellan oder Kunststoff individualisiert wird,

**dadurch gekennzeichnet, dass**

aufgrund der Abtastung und Digitalisierung eines Positivmodells (46, 47) der Situation im Patientenmund unter Berücksichtigung der Sinterschrumpfung eine vergrösserte Ausführungsform des Grundgerüsts (14) mit einer inneren und einer äusseren Oberfläche (20, 22) durch Material-abtrag aus einem Rohling (48) aus poröser Keramik herausgearbeitet wird, wobei die Steuerbefehle an eine geeignete Werkzeugmaschine zum Herausarbeiten der vergrösserten Ausführungsform des Grundgerüsts (14) aus dem Rohling (48) von der Digitalisierung zeitlich entkoppelt erfolgt.

2. Verfahren nach Anspruch 1, **dadurch gekennzeichnet, dass** ein die Situation im Patientenmund unvollständig wiedergebendes Positivmodell (46, 47) bezüglich der dreidimensionalen äusseren und/ oder inneren Oberfläche (20,22) rechentechnisch ergänzt wird, insbesondere im Bereich der Brükkenglieder (40) von Zahnbrücken (38).

3. Verfahren nach Anspruch 1 oder 2, **dadurch gekennzeichnet, dass** der Vergrösserungsfaktor ( f ) des Positivmodells (46,47) eines Grundgerüstes (46) aufgrund der Materialzusammensetzung und der Pulvereigenschaften festgelegt wird, vorzugsweise nach der Formel

$$f = 3\sqrt{\frac{\rho_S}{\rho_R}}$$

wobei $\rho_R$ das Raumgewicht des vorgefertigten Rohlings und $\rho_S$ das nach der Sinterung erreichbare Raumgewicht bedeuten.

4. Verfahren nach einem der Ansprüche 1 bis 3, **dadurch gekennzeichnet, dass** die Zahnkronen (28) und/oder Zahnbrücken (38) mit dünn auslaufender Berandung (16) ausgebildet werden.

5. Verfahren nach einem der Ansprüche 1 bis 4, **dadurch gekennzeichnet, dass** das bearbeitete, vergrösserte Grundgerüst (14) gesintert wird zu einer

Dichte $\rho_S$ von 90 bis 100% der theoretisch möglichen Dichte, vorzugsweise zu einer Dichte $\rho_S$ von 96 bis 100% der theoretisch möglichen Dichte, insbesondere zu einer Dichte $\rho_S$ von über 99% der theoretisch möglichen Dichte.

6. Verfahren nach einem der Ansprüche 1 bis 5, **dadurch gekennzeichnet, dass** ein grüner oder vorgesinterter Rohling (48) aus gepresstem, feinem Keramikpulver eingesetzt wird, wobei eine Vorsinterung vorzugsweise erst nach dem Abtragen der äussersten Materialschicht (50) erfolgt.

7. Verfahren nach einem der Ansprüche 1 bis 6, **dadurch gekennzeichnet, dass** der Rohling (48) mechanisch und/oder optisch bearbeitet wird, wobei vorzugsweise zuerst eine Rohbearbeitung, dann eine Endbearbeitung erfolgt.

8. Verfahren nach einem der Ansprüche 1 bis 7, **dadurch gekennzeichnet, dass** der Rohling (48) vor der Vorbearbeitung einer Wärmebehandlung bei Temperaturen im Bereich von 50 bis 200°C, vorzugsweise von 90 bis 150°C, unterworfen wird, für eine Zeitdauer von 2 bis 20 Stunden, vorzugsweise von 2 bis 6 Stunden.

9. Verfahren nach Anspruch 8, **dadurch gekennzeichnet, dass** der Rohling (48) nach der Wärmebehandlung materialabtragend zum vergrösserten Grundgerüst (14) weiterverarbeitet wird.

10. Verfahren nach einem der Ansprüche 1 bis 9, **dadurch gekennzeichnet, dass** der Rohling (48) vor der Weiterverarbeitung zum vergrösserten Grundgerüst (14) eine Vorsinterung für 0.5 bis 6 Stunden bei einer Temperatur von mindestens etwa 450°C erfährt, die vorzugsweise im Bereich von 600 bis 1200°C liegt.

11. Verfahren nach einen der Ansprüche 1 bis 10, **dadurch gekennzeichnet, dass** ein Rohlinge (48) aus wenigstens einem der Metalloxidpulver der Gruppe bestehend aus $Al_2O_3$, $TiO_2$, $MgO$, $Y_2O_3$ und Zirkonoxidmischkristall $Zr_{1-x}Me_xO_{2-(\frac{4n}{2})_x}$ eingesetzt wird, wobei Me ein Metall ist, das in Oxidform als zwei-, drei- oder vierwertiges Kation vorliegt (n= 2, 3, 4 und $0 \leq x \leq 1$) und die tetragonale und/oder die kubische Phase des Zirkonoxids stabilisiert.

12. Verfahren nach einem der Ansprüche 1 bis 11, **dadurch gekennzeichnet, dass** Metalloxidpulver mit einem organischen Binder eingesetzt werden, vorzugsweise aus wenigstens einer der Klassen Polyvinylalkohole (PVA), Polyacrylsäuren (PAA), Zellulosen, Polyethylenglukole und/oder Thermoplaste.

13. Verfahren nach Anspruch 12, **dadurch gekenn-**

**zeichnet, dass** der Binderanteil im Bereich 0.1 bis 45 vol% liegt, vorzugsweise im Bereich 0.1 bis 5 vol%.

14. Rohling (48) aus poröser Keramik zur Durchführung des Verfahrens nach einem der Absprüche 1 bis 13, **dadurch gekennzeichnet, dass** auf dem abzutragenden Rohling (48) selbst, seiner Verpackung, einer Anhängeetikette oder einem Beipackzettel ein maschinell oder mit menschlichen Sinnesorganen erfassbarer Informationscode (C) mit Daten zur individuellen Eingabe des kompensierenden Vergrösserungsfaktors (f) aufgebracht ist.

15. Rohling (48) nach Anspruch 14, **dadurch gekennzeichnet, dass** der Identifikationscode (C) optisch, elektromagnetisch oder mechanisch-taktil erfassbar aufgebracht ist.

**Claims**

1. Process for production of a synthetic toothless substitute (28, 38) of pressed fine ceramic powder which can fit on at least one preprepared dental stump (10), where taking into account the shrinkage the inner surface (22) of a fully ceramic skeletal structure (14) of biologically compatible material is calculated, where the geometric conditions of the patient's mouth are scanned and digitised, the data enlarged linear in all directions by a enlargement factor (f) compensating precisely for the sinter shrinkage, transferred to the control electronics of at least one processing machine and suitable tool paths derived from this, the enlarged design form of the skeletal structure (14) dense sintered to the direct end dimensions and then individualised by capping with coating material (24) of porcelain or plastic,
**characterised in that**
on the basis of the scanning and digitisation of a positive model (46, 47) of the situation in the patient's mouth, taking into account the sinter shrinkage, an enlarged design form of the skeletal structure (14) with an inner and an outer surface (20, 22) is produced by material removal from a blank (48) of porous ceramic, where the control commands are sent to a suitable machine tool for production of the enlarged design form of the skeletal structure (14) from the blank (48) temporally decoupled from the digitisation.

2. Process according to claim 1, **characterised in that** a positive model incompletely reflecting the situation in the patient's mouth (46, 47), is supplemented by computer technology in relation to the three-dimensional outer and/or inner surface (20, 22), in particular in the area of the bridge elements (40) of tooth bridges (38).

3. Process according to claim 1 or 2, **characterised in that** the enlargement factor (f) of the positive model (46, 47) of a skeletal structure (46) is established on the basis of the material composition and powder properties, preferably according to the formula

$$f = 3\sqrt{\frac{\rho_S}{\rho_R}}$$

where $\rho_R$ is the relative density of the preproduced blank and $\rho_S$ the relative density achievable after sintering.

4. Process according to any of claims 1 to 3, **characterised in that** the tooth crowns (28) and/or tooth bridges (38) are formed with fine run-out margins (16).

5. Process according to any of claims 1 to 4, **characterised in that** the machined enlarged skeletal structure (14) is sintered to a density $\rho_S$ of 90 to 100% of the theoretically possible density, preferably a density $\rho_S$ from 96 to 100% of the theoretically possible density, in particular to a density $\rho_S$ of over 99% of the theoretically possible density.

6. Process according to any of claims 1 to 5, **characterised in that** a green or presintered blank (48) of pressed fine ceramic powder is used, where presintering preferably takes place only after removal of the outer material layer (50).

7. Process according to any of claims 1 to 6, **characterised in that** the blank (48) is processed mechanically and/or optically, where preferably first a rough machining and then a final machining take place.

8. Process according to any of claims 1 to 7, **characterised in that** the blank (48) is subjected before pretreatment to a heat treatment at temperatures in the range from 50 to 200°C, preferably from 90 to 150°C, for a duration of 2 to 20 hours, preferably from 2 to 6 hours.

9. Process according to claim 8, **characterised in that** the blank (48) after heat treatment is processed further with material removal into the enlarged skeletal structure (14).

10. Process according to any of claims 1 to 9, **characterised in that** the blank (48), before further processing into the enlarged skeletal structure (14), undergoes presintering for 0.5 to 6 hours at a temperature of at least 450°C, preferably in the range from 600 to 1200°C.

**11.** Process according to any of claims 1 to 10, **characterised in that** a blank (48) of at least one of the metal oxide powders of the group consisting of $Al_2O_3$, $TiO_2$, $MgO$, $Y_2O_3$ and zircon oxide mixed crystal $Zr_{1-x}Me_xO_2-(\frac{4n}{2})_x$ is used, where Me is a metal which is present in the oxide form as a bi-, tri- or tetravalent cation (n = 2, 3, 4 and 0 < x < 1) and stabilises the tetragonal and/or cubic phase of the zircon oxide.

**12.** Process according to any of claims 1 to 11, **characterised in that** metal oxide powder with an organic bonding agent is used, preferably from at least one of the classes polyvinyl alcohols (PVA), polyacrylic acids (PAA), celluloses, polyethyleneglucols and/or thermoplastics.

**13.** Process according to claim 12, **characterised in that** the proportion of binding agent lies in the range from 0.1 to 45 vol%, preferably in the range 0.1 to 5 vol%.

**14.** Blank (48) of porous ceramic for performance of the process according to any of claims 1 to 13, **characterised in that** on the blank (48) from which the material is to be removed itself, its packing, an attachment label or a packing leaflet, is applied an information code (C) legible by machine or with human sensory organs, with data for individual input of the compensating enlargement factor (f).

**15.** Blank (48) according to claim 14, **characterised in that** the applied identification code (C) is detectable optically, electromagnetically or mechanically-tactile.

### Revendications

**1.** Procédé pour la fabrication d'une prothèse de dent artificielle (28,38) qui peut s'adapter sur un moignon dentaire (10) qui a subi une préparation préalable et qui est constituée de poudre de céramique fine comprimée, procédé dans lequel, en prenant en compte le retrait, on calcule la surface intérieure (22) d'une structure de base (14) réalisée entièrement en céramique à partir d'une matière compatible du point de vue biologique, on explore et on numérise les relations géométriques de la bouche du patient, on augmente les données de manière linéaire dans toutes les directions d'un facteur d'agrandissement (f) qui compense le retrait de frittage de manière exacte, on transfère les données dans l'électronique de commande d'au moins une machine de traitement et on en déduit des trajets d'outil appropriés, on réalise un frittage à la densité maximale de la forme de réalisation agrandie de la structure de base (14) sur la masse finale directe et on individualise ensuite ladite forme de réalisation de la structure de base (14) au moyen d'un parement avec une matière de revêtement (24) en porcelaine ou en matière synthétique,

**caractérisé en ce que**

sur la base de l'exploration et de la numérisation d'un modèle en positif (46, 47) de la situation dans la bouche du patient, en prenant en compte le retrait du frittage, on fabrique une forme de réalisation agrandie de la structure de base (14) qui comporte une surface extérieure (20) et une surface intérieure (22) par enlèvement de matière à partir d'une ébauche (48) en matière céramique poreuse, les ordres de commande sont envoyés à une machine-outil appropriée pour la fabrication de la forme de réalisation agrandie de la structure de base (14) à partir de l'ébauche (48) avec un décalage dans le temps par rapport à la numérisation.

**2.** Procédé selon la revendication 1, **caractérisé en ce que** l'on complète, par une technique de calcul, un modèle en positif (46, 47) qui reproduit de manière incomplète la situation dans la bouche du patient en ce qui concerne les surfaces extérieure (20) et/ou intérieure (22) en trois dimensions, en particulier dans la zone des éléments de pontage (40) de bridges dentaires (38).

**3.** Procédé selon la revendication 1, **caractérisé en ce que** le facteur d'agrandissement (f) du modèle en positif (46, 47) d'une structure de base (46) est déterminé sur la base de la constitution chimique de la matière et des caractéristiques de la poudre, de préférence selon la formule suivante :

$$f = 3\sqrt{\frac{\rho_S}{\rho_R}}$$

dans laquelle $\rho_R$ est le poids spécifique de l'ébauche préfabriquée et $\rho_S$ est le poids spécifique qui peut être obtenu après le frittage.

**4.** Procédé selon l'une quelconque des revendications 1 à 3, **caractérisé en ce que** les couronnes dentaires (28)et/ou les bridges dentaires (38) sont réalisés avec une bordure (16) qui se termine avec une faible épaisseur.

**5.** Procédé selon l'une quelconque des revendications 1 à 4, **caractérisé en ce que** la structure de base (14) traitée et agrandie est frittée à une densité $\rho_S$ comprise entre 90 et 100 % de la densité théorique possible, de préférence à une densité $\rho_S$ comprise

entre 96 et 100 % de la densité théorique possible, en particulier à une densité $\rho_S$ supérieure à 99% de la densité théorique possible.

6. Procédé selon l'une quelconque des revendications 1 à 5, **caractérisé en ce que** l'on utilise une ébauche (48) brute ou ayant subi un préfrittage et constituée de poudre céramique fine compressée, un préfrittage étant effectué de préférence seulement après l'enlèveme de la couche de matière extérieure (50).

7. Procédé selon l'une quelconque des revendications 1 à 6, **caractérisé en ce que** l'ébauche (48) est traitée mécaniquement et/ou optiquement, en réalisant de préférence tout d'abord un dégrossissage puis une finition.

8. Procédé selon l'une quelconque des revendications 1 à 7, **caractérisé en ce que** l'ébauche (48) est soumise, avant le traitement préalable, à un traitement thermique à des températures comprises entre 50 et 200 °C, de préférence entre 90 et 150 °C, pendant une durée comprise entre 2 et 20 heures, de préférence entre 2 et 6 heures.

9. Procédé selon la revendication 8, **caractérisé en ce que**, après le traitement thermique, l'ébauche (48) subit un traitement ultérieur par enlèvement de matière pour obtenir la structure de base (14) agrandie.

10. Procédé selon l'une quelconque des revendications 1 à 9, **caractérisé en ce que**, avant le traitement ultérieur par enlèvement de matière pour obtenir la structure de base (14) agrandie, l'ébauche (48) est soumise à un préfrittage pendant 0,5 à 6 heures, à une température d'au moins environ 450 °C qui, de préférence, se trouve comprise entre 600 et 1 200 °C.

11. Procédé selon l'une quelconque des revendications 1 à 10, **caractérisé en ce que** l'on utilise une ébauche (48) constituée d'au moins une poudre d'oxyde métallique du groupe comprenant $Al_2O_3$, $TiO_2$, MgO, $Y_2O_3$ et d'un cristal de mélange d'oxyde et de zirconium $Zr_{1-x}Me_xO_2(\frac{4n}{2})_x$, dans lequel Me est un métal qui se trouve sous la forme d'un oxyde en tant que cation de valence 2, 3 ou 4 (n = 2, 3, 4 et x compris entre 0 et 1) et stabilise la phase tétragonale et/ou cubique de l'oxyde de zirconium.

12. Procédé selon l'une quelconque des revendications 1 à 11, **caractérisé en ce que** l'on utilise des poudres d'oxyde métallique comprenant un liant organique constitué, de préférence, d'au moins une des classes des alcools de polyvinyle (PVA), des acides polyacryliques (PAA), des celluloses, des polyéthylèneglycols et/ou des matières thermoplastiques.

13. Procédé selon la revendication 12, **caractérisé en ce que** la teneur en liant est comprise entre 0,1 et 45 % en volume, de préférence entre 0,1 et 5 % en volume.

14. Ebauche (48) en matière céramique poreuse pour la mise en oeuvre du procédé selon l'une quelconque des revendications 1 à 13,
   **caractérisée en ce que**
   on place sur l'ébauche (48) devant subir l'enlèvement de matière elle-même, sur son emballage, sur une étiquette qui y est accrochée ou sur une notice, un code d'informations (C) qui peut être saisi par une machine ou par les organes sensoriels humains et qui comporte des données pour l'entrée séparée du facteur d'agrandissement (f) de compensation.

15. Ebauche (48) selon la revendication 14, **caractérisée en ce que** le code d'informations (C) peut être saisi de manière optique, électromagnétique ou mécaniquement par effet tactile.

Fig.1

Fig.2

**Fig.3**

**Fig.4**

**Fig.5**

Fig.6

Fig.7

Fig.8

Fig.9